(19) Europäisches Patentamt
European Patent Office
Office européen des brevets

(11) EP 1 659 108 B1

(12) **EUROPEAN PATENT SPECIFICATION**

(45) Date of publication and mention of the grant of the patent:
**16.07.2008 Bulletin 2008/29**

(21) Application number: **05025458.0**

(22) Date of filing: **22.11.2005**

(51) Int Cl.:
*C07C 67/343* (2006.01)
*C07C 51/347* (2006.01)
*C07C 57/04* (2006.01)
*C07B 37/04* (2006.01)
*C07C 69/24* (2006.01)
*C07C 69/54* (2006.01)
*C07C 53/124* (2006.01)

(54) **Two-step process to produce methyl branched organic compounds using dimethyl ether and hydrogen**

Zwiestufiges Verfahren zur Herstellung von methylverzweigten organischen Verbindungen unter Verwendung von Dimethylether und Wasserstoff

Procédé à deux étapes pour la préparation de composés organiques à ramifications méthyle utilisant l'ether dimethylique et hydrogène

(84) Designated Contracting States:
**AT BE BG CH CY CZ DE DK EE ES FI FR GB GR HU IE IS IT LI LT LU LV MC NL PL PT RO SE SI SK TR**

(30) Priority: **23.11.2004 US 996075**

(43) Date of publication of application:
**24.05.2006 Bulletin 2006/21**

(73) Proprietor: **Air Products and Chemicals, Inc.**
**Allentown, PA 18195-1501 (US)**

(72) Inventors:
- **Waller, Francis Joseph**
  **Allentown, PA 18103-9670 (US)**
- **Brown, Dennis Mackenzie**
  **Fogelsville, PA 18051 (US)**

(74) Representative: **Kador & Partner**
**Corneliusstrasse 15**
**80469 München (DE)**

(56) References cited:
**US-B1- 6 329 549**

- **R.T. MORRISON AND R.N. BOYD: "Organic Chemistry, 4th Ed." 1983, ALLYN AND BACON INC. , XP002371880 * page 831 ***

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 659 108 B1

## Description

BACKGROUND OF THE INVENTION

This invention relates to the production of a saturated hydrocarbon containing at least one new methyl branch by methylating a carbon adjacent to an electron withdrawing group (EWG) using dimethyl ether as the source of the methyl group and hydrogenating the EWG. In particular, this invention is useful in a refinery operation where there are olefins with low octane number. Methylating with dimethyl ether and hydrogenating the olefins produces saturated hydrocarbons with methyl branches which have higher octane value.

Chemical processes that allow a refinery to increase octane number without the addition of oxygenated blending components such as methyl t-butyl ether (MTBE) are in more demand today as MTBE is being phased out because of environmental concerns.

One approach to methylate an alpha-carbon is to form an enolate, that is, removing the proton on a carbon adjacent to an EWG with a base to form a carbanion. When the EWG contains a carbonyl group the combination of the carbanion and the carbonyl is an enolate. In organic chemistry methylation of the enolate is done with a methyl halide such as methyl iodide.

Another approach reacts intermediates, such as propionic acid, propionic acid anhydride, or methyl propionate with formaldehyde or formaldehyde dimethylacetal or trioxane or paraformaldehyde to form methacrylic acid or methyl methacrylate. Although formaldehyde can be added in excess, the preferred modes of operation utilize the carboxylic acid intermediates in excess to avoid significant yield loss due to formaldehyde side reactions in the gas phase. This approach requires the hydrogenation of methacrylic acid or methyl methacrylate to isobutyric acid and methyl isobutyrate followed by further hydrogenation of the EWGs, $CO_2H$ and $CO_2CH_3$ respectively.

For example, in U.S. Patent No. 4,736,062, Hagen et al. disclose a process of producing an alpha, beta-ethylenically unsaturated monocarboxylic acid compound which comprises the aldol-type condensation of a saturated aliphatic monocarboxylic acid and formaldehyde under vapor phase conditions in the presence of a hydrocarbon of 6 to 12 carbon atoms and a solid catalyst. This solid acid catalyst is described as comprising a cation of Group I or Group II metal and a silica support.

In U.S. Patent No. 4,761,393, Baleiko et al. describe an *in situ* method for preparing an alkali metal ion-bearing particulate siliceous catalyst suitable for enhancing the vapor-phase condensation of a gaseous, saturated carboxylic acid with formaldehyde.

In U.S. Patent No. 4,801,571 Montag et al. disclose a mixed oxide $SiO_2$-$SnO_2$-Cs ion catalyst and process for production of an alpha, beta-ethylenically unsaturated monocarboxylic acid by condensation of a saturated monocarboxylic acid with formaldehyde.

In U.S. Patent No. 4,845,070, Montag describes a catalyst suitable for production of methacrylic acid by condensation of propionic acid with formaldehyde. The catalyst comprises a support which consists essentially of porous silica gel with cesium ions on the catalyst support surface, this support surface having a surface area of about 50 to about 150 $m^2$/g, a porosity of less than about 1$cm^3$/gm, a pore size distribution such that less than about 10 percent of the pores present in the catalyst have a pore diameter greater than about 750 angstroms, and the cesium ions present in an amount of about 4 to about 10 percent by weight of the said catalyst.

In U.S. Patent No. 4,942,258, Smith discloses a process for regeneration of a catalyst which comprises a support which consists essentially of porous silica with cesium ions on the catalyst support surface, said catalyst useful for production of methacrylic acid by condensation of propionic acid with formaldehyde.

In U.S. Patent No. 5,710,328, Spivey et al. disclose a process for the preparation of alpha,beta-unsaturated carboxylic acids and the corresponding anhydrides which comprises contacting formaldehyde or a source of formaldehyde with a carboxylic anhydride in the presence of a catalyst comprising mixed oxides of vanadium and phosphorous, and optionally containing a third component selected from titanium, aluminum, or preferably silicon. In Ind. Eng. Chem. Res., Vol. 36, No.11, 1997, 4600-4608, Spivey et al. report that the highest yields of methacrylic acid were obtained with the Vanadium-Silicon-Phosphorous ternary oxide catalyst with V-Si-P atomic ratio of 1:10:2.8.

In U.S. Patent No. 5,808,148, Gogate et al. disclose a process for the preparation of alpha,beta-unsaturated carboxylic acids and esters which comprises contacting formaldehyde or a source of formaldehyde with a carboxylic acid, ester, or a carboxylic acid anhydride in the presence of a catalyst comprising an oxide of niobium. The optimum catalyst in the catalytic synthesis of methacrylates comprised a mixed niobium oxide-silica composition containing 10% $Nb_2O_5$ (Ind. Eng. Chem. Res., Vol. 36, No.11, 1997, 4600-4608; Symposium Syngas Conversion to Fuels and Chemicals, Div. Pet. Chem., Inc., 217 th National Meeting, American Chemical Society, Anaheim, CA, 1999, 34-36).

In a related approach to synthesizing methyl methacrylate, the synthesis of isobutyric acid is followed by oxidative dehydrogenation to yield methacrylic acid, which is then esterified with methanol to yield methyl methacrylate. The key technical challenge lies in the selective oxidative dehydrogenation of isobutyric acid to methacrylic acid and three classes of catalysts have been disclosed: 1) iron phosphates, 2) vanadium - phosphorous mixed oxides or with a

ternary component, and 3) heteropolyacids based on phosphomolybdic acid.

In Catalysis Reviews, Sci. Eng. 40(1&2), 1-38, (1998), Millet presents a comprehensive review of iron phosphate catalysts disclosed in the patent literature. According to Millet, the optimum catalysts for this process have P/Fe ratio greater than 1.0, are promoted with alkali metals, silver or lead, and may be supported on silica or alundum. The reaction is conducted at 365˚ to 450˚C in the presence of oxygen and a co-feed of up to 12 moles $H_2O$ per mole isobutyric acid is needed to generate a catalyst with high activity. In Applied Catalysis A: General, 109 (1994) 135-146, Ai et al. further discussed the role of many different promoters for iron phosphate catalyst and found that the best performance was obtained with $Pb^{2+}$.

In Journal of Catalysis 98, 401-410 (1986), Ai found that $V_2O_5$-$P_2O_5$ binary oxide catalysts were effective for the synthesis of methacrylic acid by oxidative dehydrogenation of isobutyric acid. The selectivity to methacrylic acid was a maximum for catalysts with P/V ratio in the range 1.0 to 1.6 when tested in the temperature range 190˚C to 280˚C. Ai also disclosed that these catalysts are selective in the vapor phase aldol condensation of (1) formaldehyde with propionic acid to produce methacrylic acid (Appl. Catal., 36 (1988) 221-230; J. Catal. 124, (1990) 293-296) and (2) formalin with acetic acid to produce acrylic acid (J. Catal. 107, (1987) 201-208).

In Journal of Catalysis 124 (1990) 247-258, Watzenberger et al. describe the oxydehydrogenation of isobutyric acid with heteropolyacid catalysts, such as $H_5PMO_{10}V_2O_{40}$.

In U.S. Patent No. 4,442,307, Lewis et al. disclose a process for the preparation of formaldehyde by oxidizing dimethyl ether in the presence of a catalyst comprising oxides of bismuth, molybdenum and iron. Table 1 of said patent provides the only illustrative Examples at 500˚C in which a 54%Bi-24%Mo-2%Fe catalyst afforded 42% conversion and 46% formaldehyde selectivity while a 55%Bi-25%Mo catalyst gave 32% conversion and 28% formaldehyde selectivity.

Selective catalytic C-C bond formation on MgO to produce alpha, beta-unsaturated compounds was described by Korukawa et al. (Heterogeneous Catalysis and Fine Chemicals, Guisnet et al. Eds., Elsevier Science Publishers, 1988, 299-306). The authors claim to have developed a novel synthetic route by using MeOH as a methylenylating agent. The synthetic method uses magnesium oxide catalysts activated by transition metal cations to produce formaldehyde. According to the authors, "methyl or methylene groups at alpha-position of saturated ketones, esters or nitriles are converted to vinyl groups by the C-C bond formation using methanol as a $CH_2$= source." The reaction of methyl propionate with methanol over manganese-promoted MgO afforded 10% conversion of methyl propionate and produced 60% methyl methacrylate (MMA), 18% methyl isobutyrate (MIB) and 22% ketones. The reaction occurs at 400˚C in the absence of $O_2$ and co-produces $H_2$ and $H_2O$.

In U.S. Patent No. 3,845,155, Heckelsberg discloses a process to alkylate olefins to higher olefins with an alcohol or dialkyl ether. Table 1 of said patent provides the only illustrative Examples in which butene-2 and dimethyl ether are converted to $C_5$ and $C_5$ products with eta-alumina and zirconia catalysts. The exact structure of the $C_5$ and $C_5$ products are not mentioned. This patent is directed to the preparation of olefins, and does not disclose or suggest hydrogenating any unsaturated groups.

In our prior U.S. Patent No. 6,329,549, we disclosed a process comprising the use of dimethyl ether to introduce a methyl group or carbon-carbon double bond on a carbon adjacent to an EWG in the presence of a particular group of catalysts. This patent does not disclose hydrogenating the EWG to provide a reduced EWG, but rather, teaches dehydrogenating the methyl group adjacent to the EWG to provide an alpha, beta-unsaturated compound.

EP 0111605, Grasselli et al., discloses a process for the production of unsaturated acids and esters comprising reacting in the vapor phase at a temperature of 200˚C to 500˚C a first reactant selected from saturated monocarboxylic acids, esters and anhydrides, a second reactant selected from primary and secondary alcohols and dialkyl ethers, and oxgyen, in the presence of an oxidation catalyst, said catalyst having at least two elements, at least one element being a multi-valent metallic element. This document is directed to the preparation of unsaturated compounds, and does not disclose or suggest hydrogenating any unsaturated groups.

Despite the foregoing developments, it is desired to provide a process comprising methylating a carbon adjacent to an EWG and hydrogenating the EWG. It is further desired to provide such a process, comprising the use of dimethyl ether to introduce a methyl group on a carbon adjacent to an EWG, and the use of hydrogen to hydrogenate the EWG. It is still further desired to provide a two-step chemical process that converts a molecule containing an alpha-carbon adjacent to an olefin to a saturated hydrocarbon containing additional methyl branches for octane value.

All references cited herein are incorporated herein by reference in their entireties.

BRIEF SUMMARY OF THE INVENTION

Accordingly, the invention provides a process for providing a methyl group on an alpha-carbon adjacent to a hydrogenated electron withdrawing group, said process comprising:

providing a molecule containing the alpha-carbon and an electron withdrawing group;
reacting the molecule in a presence of an acid catalyst with dimethyl ether to substitute the methyl group on the

alpha-carbon; and

hydrogenating the electron withdrawing group to provide the hydrogenated electron withdrawing group adjacent to the alpha-carbon substituted with the methyl group.

## DETAILED DESCRIPTION OF THE INVENTION

The process of the invention uses dimethyl ether to methylate an alpha-carbon adjacent to an EWG. The EWG is then reduced with hydrogen over a hydrogenation catalyst in another process step to form a saturated compound containing a reduced electron withdrawing group (REWG) with at least one new methyl group (or branch). The preferred process of the invention is therefore a two-step process, wherein the first step comprises methylating the alpha-carbon and the second step comprises hydrogenating the electron withdrawing group adjacent to the methylated alpha-carbon.

The subject invention can be successfully applied to feedstocks of intermediate compounds which contain various EWGs. Preferred embodiments of said compounds can be represented by Formula I when the EWG is a terminal group:

$$R'(CH_2)_nCH_2\text{-EWG} \qquad \text{(Formula I)}$$

where R' is H when n is 0 to 18, otherwise R' is alkyl, alkene, EWG or aryl, and by Formula II when the EWG is an internal group:

$$R'(CH_2)_nCH_2\text{-EWG-}CH_2(CH_2)_mR'' \qquad \text{(Formula II)}$$

where R' is H when n is 0 to 9 and alkyl, alkene, EWG or aryl when n > 9, and R" is H when m is 0 to 9 and alkyl, alkene, EWG or aryl when m > 9.

The invention is not limited to Formulae I and II. The use of intermediate compounds represented by other formulae is also within the scope of the invention. The key element, $-CH_2$-EWG or $-CH_2$-EWG-, can be located in different places within the compounds.

General classes of intermediate compounds suitable for use in the invention include, but are not limited to, carboxylic acids, carboxylic acid esters, nitriles, aromatic ring, alkenes, ketones and aldehydes. Specific, non-limiting, examples include acetic acid, propionic acid, methyl acetate, methyl propionate, acetonitrile, propionitrile, acetone, propionaldehyde and other compounds containing these structural units. For an alkene, non-limiting examples include cyclic or acyclic compounds, such as propylene, 2-butene, isobutene, 1,3-butadiene, 3-methyl-1-butene, 2-methyl-2-butene, isoprene, 2-pentene, 2,3-dimethyl-2-butene, cyclopentadiene, 2-ethyl-1-hexene, 4-octene, cyclooctene, 1-decene, 2-decene, 2-eicosene, and the like, and mixtures thereof. Particularly advantageous results are obtained with $C_4$ to $C_{10}$ acyclic monoolefins.

The intermediate compound(s) are provided in a feedstock, which is combined with the reactant(s). Feedstocks can consist essentially of at least one intermediate compound, or can comprise at least one intermediate compound plus additional components. In certain embodiments, the feedstock contains the intermediate compound(s) diluted with paraffins, such as in a number of olefinic refinery streams.

The intermediate compound is reacted with dimethyl ether to add at least one methyl group to the alpha-carbon adjacent to the EWG of the intermediate compound. This first process step is preferably catalyzed, most preferably with the catalyst system taught in our earlier U.S. Patent No. 6,329,549 B1. Thus, suitable catalysts include, but are not limited to, partial oxidation catalyst functionalities, and particularly Lewis acid catalysts, combined Lewis acid and Brönsted acid catalysts, and Lewis acid or mixed Lewis plus Brönsted acids containing selected partial oxidation catalyst property. Preferred catalysts include, but are not limited to, gamma-alumina, amorphous silica-alumina, steam-treated zeolites such as ultra-stable Y, acid washed clays, alumina impregnated clays, and $MoO_3$ on gamma-alumina.

The ideal ratio of dimethyl ether to intermediate compound(s) (DME:IC ratio) can be selected by conventional stoichiometric calculations supplemented by routine experimentation using the present disclosure as a guide. In certain embodiments, the DME:IC ratio ranges from about 0.5 to about 20.

Generally, reaction temperatures for the first process step range from about 150˚C to 500˚C, but are preferably from 250˚C to 400˚C. When the EWG is an olefin, the reaction temperature range is preferably from 25˚C to 150˚C and more preferably from 25˚C to 125˚C.

Reaction pressures for the first process step may vary, but typically range from 0 to 50 psig. Total feed space velocities vary from about 100 to 5000 $hr^{-1}$, preferably 200 to 2000 $hr^{-1}$. Gas Hourly Space Velocity (GHSV) is defined as the total feed rate in $cm^3$ gas at STP/hr ratioed to the catalyst bed volume in $cm^3$. The resulting conversion of DME generally will range from about 10% to 80% with total selectivity to all desirable products greater than 60%.

The first process step is preferably conducted in the absence of an oxidant, or at least in the absence of an amount of oxidant sufficient to form alpha, beta-unsaturated compounds. However, it is within the scope of less preferred

embodiments of the invention to perform the first process step in the presence of an amount of oxidant sufficient to form alpha, beta-unsaturated compounds, and to subsequently hydrogenate these compounds.

The intermediate product of the preferred first process step can be represented by either of the following molecular formulas:

$R'(CH_2)_nCH(CH_3)\text{-EWG}$ (Formula III)

$R'(CH_2)_nCH(CH_3)\text{-EWG-}CH(CH_3)(CH_2)_mR''$ (Formula IV)

where R', R", n and m are as defined above for Formulas I and II, respectively. Thus, certain embodiments of the first process step can be represented by Equation I (when the EWG is a terminal group, as in Formulas I and III) or Equation II (when the EWG is an internal group, as in Formulas II and IV), as shown below:

(a) Eq. I

$$R'(CH_2)_nCH_2\text{-EWG} + CH_3OCH_3 \rightarrow R'(CH_2)_nCH(CH_3)\text{-EWG} + CH_3OH$$

(b) Eq. II

$$R'(CH_2)_nCH_2\text{-EWG-}CH_2(CH_2)_mR'' + 4CH_3OCH_3 \rightarrow R'(CH_2)_nCH(CH_3)\text{-EWG-}CH_2(CH_2)_mR'' + R'(CH_2)_nCH_2\text{-EWG-}CH(CH_3)(CH_2)_mR'' + R'(CH_2)_nCH(CH_3)\text{-EWG-}CH(CH_3)(CH_2)_mR'' + 4CH_3OH$$

where EWG, R', R", n and m are as defined above for Formulas I and II, respectively. Specific, non-limiting, examples of the reaction of the first process step include the formation of propionic acid from acetic acid, isobutyric acid from propionic acid, methyl propionate from methyl acetate, methyl isobutyrate from methyl propionate, propionitrile from acetonitrile, isobutyronitrile from propionitrile, 3-methyl-1-butene from 1-butene and methyl ethyl ketone from acetone.

In certain embodiments, a methyl group is added from dimethyl ether to the alpha-carbon of an alkene to produce the corresponding methylated olefin compound. Specific, non-limiting, examples include the formation of 2-pentene from 2-butene, 4-methyl-2-pentene from 2-pentene, 3,6-dimethyl-4-octene from 4-octene and 4-methyl-2-decene from 2-decene. Other methylated olefin compounds are possible for 2-butene, 2-pentene, 4-octene and 2-decene.

In the second process step of the invention, the intermediate product(s) of the first process step are hydrogenated. As used herein, the term "hydrogenation" denotes the addition of at least two hydrogens to a functional group capable of being hydrogenated. Hydrogenation therefore reduces the EWG to a REWG. Specific, non-limiting, examples of the hydrogenation reaction of the second process step are -CN to a primary amine, $-CH_2NH_2$, -COR to -CHOHR, $-CH{=}CH_2$ to $-CH_2CH_3$, $-CO_2R$ to $-CH_2OH$, -CH=CH- to $-CH_2CH_2-$ Table 1 lists these and other preferred examples of EWGs and their corresponding REWGs.

Table 1. Electron Withdrawing Group Hydrogenation

| EWG | REWG |
|---|---|
| $-CO_2H$ | $-CH_2OH$ |
| $-CO_2R$ | $-CH_2OH$ |
| —COR | —CHOHR |
| —CN | $-CH_2NH_2$ |
| —C≡CR | $-CH_2CH_2R$ |
| 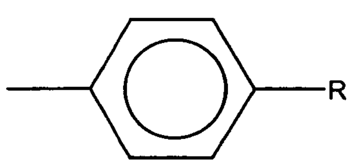 | 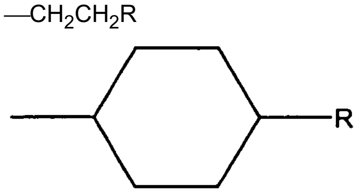 |
| —CH≡CHR | $-CH_2CH_2R$ |

To facilitate the hydrogenation reaction, a hydrogenation catalyst is preferably used. Different types of catalysts are used depending upon the functional group to be reduced. The catalysts can be homogeneous (soluble in the reaction medium) or heterogeneous (solid). Non-limiting examples of suitable catalysts are summarized in Table 2.

Table 2. Catalysts for Hydrogenation of EWG

| EWG | REWG | Catalysts |
|---|---|---|
| $—CO_2H$ | $—CH_2OH$ | Cu-chromium oxide; Cu-Ba-Cr oxide; $R_2O_7$ |
| $—CO_2R$ | $—CH_2OH$ | Cu-Cr oxide; Cu-Ba-Cr oxide |
| —COR | —CHOHR | Raney Ni; Ni-kieselguhr; Pt metals in ethanol |
| —CN | $—CH_2NH_2$ | Pd on C; Raney Ni |
| —C≡CR | $—CH_2CH_2R$ | Raney Ni; Pd on C |
| 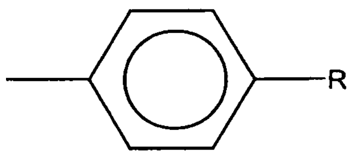 | 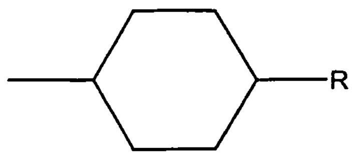 | Ni-ieselguhr; Raney Ni; Pt oxide; Rh-Pt oxide |
| —CH=CHR | $—CH_2CH_2R$ | Adams Pt oxide; Raney Ni; Pd on C; PdO; Nickel boride |

Additional guidance regarding the selection and use of hydrogenation catalysts can be found in, e.g., Nishimura, Handbook of Heterogeneous Catalytic Hydrogenation for Organic Synthesis, John Wiley and Sons, Inc., 2001.

The reaction conditions for each catalyst are different depending upon the hydrogenation of EWG to REWG. The person skilled in the art could take the Nishimura handbook and by consulting the many references find reaction conditions for the intermediate compounds or similar intermediate compounds of particular interest.

Certain embodiments of the hydrogenation reaction of the invention are described by Equation III (for terminal EWGs) or Equations IV-VI (for internal EWGs):

(a) Eq. III

$$R'(CH_2)_nCH(CH_3)\text{-EWG} + H_2 \rightarrow R'(CH_2)_nCH(CH_3)\text{-REWG}$$

(b) Eq. IV

$$R'(CH_2)_nCH(CH_3)\text{-EWG-}CH(CH_3)(CH_2)_mR'' + H_2 \rightarrow R'(CH_2)_nCH(CH_3)\text{-REWG-}CH(CH_3)(CH_2)_mR''$$

(c) Eq. V

$$R'(CH_2)_nCH(CH_3)\text{-EWG-}CH_2(CH_2)_mR'' + H_2 \rightarrow R'(CH_2)_nCH(CH_3)\text{-REWG-}CH_2(CH_2)_mR''$$

(d) Eq. VI

$$R'(CH_2)_nCH_2\text{-EWG-}CH(CH_3)(CH_2)_mR'' + H_2 \rightarrow R'(CH_2)_nCH_2\text{-REWG-}CH(CH_3)(CH_2)_mR''$$

where EWG, R', R", n and m are as defined above for Formulas I and II.

When the EWG is an olefin and the olefin is in the carbon chain, it should be understood that the olefin besides being di-substituted (as shown in certain of the Formulas and Equations above) can also be tri-and tetra-substituted with other hydrocarbon $R'''(CH_2)_pCH_2$ and/or $R''''(CH_2)_qCH_2$ groups, wherein R'" is H when p is 0 to 9 and alkyl, alkene, EWG or aryl when p > 9, and R"" is H when q is 0 to 9 and alkyl, alkene, EWG or aryl when q > 9. Specific, non-limiting, examples of suitable tri- and tetra-substituted intermediate compounds are 2-methyl-2-butene, 2,3-dimethyl-2-butene and other appropriate substituted olefins.

Various methods are available to help the researcher to draw up a list of all the reactions that are possible to tabulate for the methylation of a carbon adjacent to an EWG using dimethyl ether as the source of the methyl group and hydrogenating the EWG when the EWG is an olefin. One such method is the calculation of the Gibbs energy change. At the temperature where $\Delta G_R$=O, the equilibrium constant $K_R$ for the reaction equals unity, indicating that the reaction will progress to a considerable extent toward completion. As $\Delta G_R$ takes on more positive values, the reaction becomes less and less favored, until the yield of product shrinks to the level where the reaction is no longer of interest. When $\Delta G_R$ is negative, the reaction becomes more and more favored. The calculation of $\Delta G_R$ is from the following equation

$$\Delta G_R = \Sigma\Delta G_F\text{ (products)} - \Sigma\Delta G_F\text{ (reactants)}$$

where $\Delta G_F$ is the Gibbs free energy of formation at a particular temperature. All values are expressed in kcal/mole and

can be found in Stull et al. (The Chemical Thermodynamics of Organic Compounds, John Wiley and Sons, Inc., 1969).

One example is illustrated here and in Tables 3 and 4.

$$CH_3CH{=}C(CH_3)_2 + 2CH_3OCH_3 \rightarrow CH_3CH{=}C(Et)(CH_3) + (CH_3)_2C{=}CHEt + 2CH_3OH$$

(3-methyl-2-pentene) (2-methyl-2-pentene)

$$\Delta G_R=(2\Delta G_F(\text{MeOH})+\Delta G_F(\text{3-methyl-2-pentene})+\Delta G_F(\text{2-methyl-2-pentene}))-(\Delta G_F(\text{2-methyl-2-butene})+2\Delta G_F(\text{dimethylether}))$$

at 27°C, $\Delta G_R$= -6.52 kcal/mole
at 127°C, $\Delta G_R$= +1.31 kcal/mole

Table 3. Reaction of 2-Methyl-2-Butene with Dimethyl Ether

| RxnTemp (°C) | $\Delta G_R$(kcal/mole) | Conclusion |
|---|---|---|
| 27 | -6.52 | reaction proceeds as written |
| 127 | +1.31 | reverse reaction more favorable |

These calculations demonstrate that the reaction of dimethyl ether with 2-methyl-2-butene is favored somewhere between 27 to 127°C. In fact, the reaction proceeds better at temperatures closer to room temperature with the appropriate catalyst.

Likewise the same $\Delta G_R$ for hydrogenation can be calculated, as follows:

$$CH_3CH{=}C(Et)(CH_3) + (CH_3)_2C{=}CHEt + 2H_2 \rightarrow CH_3CH_2CH(Et)(CH_3) + CH_3CH(CH_3)CH_2CH_2CH_3$$

(3-methyl pentane) (2-methyl pentane)

$$\Delta G_R=(\Delta G_F(\text{3-methyl pentane}) + \Delta G_F(\text{2-methyl pentane}))-(\Delta G_F(\text{3-methyl-2-pentene})+\Delta G_F(\text{2-methyl-2-pentene})+2\Delta G_F(H_2))$$

at 27°C, $\Delta G_R$ = -32.91 kcal/mole
at 127°C, $\Delta G_R$ = -26.58 kcal/mole

Table 4. Reaction of Methylated 2-Methyl-2-Butene with Hydrogen

| Rxn Temp(°C) | $\Delta G_R$ (kcal/mole) | Conclusion |
|---|---|---|
| 27 | -32.91 | reaction proceeds as written |
| 127 | -26.58 | reaction proceeds as written |
| 227 | -20.14 | reaction proceeds as written |

These calculations demonstrate that the hydrogenation of the reaction products from the methylation of a carbon adjacent to an EWG proceeds over a broader temperature range from 27 to 227°C.

The invention will be illustrated in more detail with reference to the following Examples, but it should be understood that the present invention is not deemed to be limited thereto.

EXAMPLES

The process for carrying out the methylation reactions is similar to the processes used in the prior art, except for (among other things) the substitution of dimethyl ether for formaldehyde and the preferred catalysts of this invention. Catalyst performance was determined using a down-flow, heated packed bed reactor system. The reactor tube was 0.5" (1.3 cm) o.d. with a 0.049" (0.12 cm) wall thickness. To ensure that a single vapor phase feed was passed through the catalyst bed the liquid feed, as well as the DME, air, and nitrogen co-feeds were all pre-heated by passing each feed through a length of coiled 0.125" (0.32 cm) o.d. tubing heated and maintained at 200˚C. Further, the feeds were combined and mixed in the top zone of the reactor tube which contained inert quartz chips. Typically 5.0 $cm^3$ of 20-35 mesh (Tyler Equivalent) of catalyst particles was loaded in the reactor tube which contained a centrally located thermocouple. The catalyst bed was supported in the reactor tube on a small wad of quartz wool followed by more quartz chips which completely filled the tube. The entire reactor tube fit concentrically and snugly into a solid stainless steel block which is heated to maintain a constant temperature zone. The effluent from the reactor was carried in heat traced 0.0625" (0.16 cm) tubing and maintained at 200˚C. The reactor pressure was not regulated but was typically between 7 to 10 psig. Samples were analyzed on-line by injecting a 250 microliter gas sample at 180˚C onto a HP 5890 Gas Chromatograph. Organic products were determined using a flame-ionization detector, while inorganic compounds were determined on a thermal conductivity detector. Both detectors were calibrated by molar response factors and $N_2$ was used as an internal standard.

The process of the present invention preferably takes place in the gas (or vapor) phase. However, embodiments of the invention can be conducted in the liquid or slurry phase.

The following parameters are useful to define the process of the invention:

$$\text{Gas Hour Space Velocity (GHSV)} = cm^3 \text{ feed (STP)}/cm^3 \text{ catalyst/hr} = hr^{-1};$$

$$\%\ \text{PA Conversion} = 100 \times (PA_{in} - PA_{out})/PA_{in},$$

where $PA_{in}$ is the mols of PA in the inlet, and
$PA_{out}$ is the mols of PA in the outlet;

$$\%\ \text{DME Conversion} = 100 \times (DME_{in} - DME_{out})/DME_{in},$$

where $DME_{in}$ is the mols of DME in the inlet, and
$DME_{out}$ is the mols of DME in the outlet;

$$\%\ \text{Carbon Balance} = 100 \times \frac{\text{(total moles carbon analyzed in effluent)}}{(3 \times \text{moles } PA_{in} + 2 \times \text{moles } DME_{in})}$$

For a particular component analyzed in the effluent, the carbon in that component which is derived from PA is used to determine PA-based selectivity. Therefore, the PA-based selectivity (%S(PAB)) is determined as follows:

$$\%S(PAB) = 100 \times \frac{\text{(moles carbon for component)} \times \text{(multiplier)}}{(3 \times \text{moles PA consumed})}$$

Table 5 below shows the carbon accounting used and gives the "multiplier" for determining the PA-based selectivity. The multiplier (M) is defined as follows:

$$M = \text{(No. Carbons in PA molecule)/(Carbons in component from PA)}$$
$$= 3\text{/(Carbons in component from PA)}$$

Thus, for acetaldehyde, the "multiplier" is 3/2 or 1.500. For methyl isobutyrate, the multiplier is 3/5 or 0.600 since two of the carbons in the molecule are derived from DME.

Examples 1 and 2 (*gamma-$Al_2O_3$*)

Five experiments were conducted using the above described procedure to evaluate gamma-$Al_2O_3$ in the synthesis of methyl methacrylate by oxidative dehydrogenation of propionic acid and dimethyl ether. A sample of 1/8" gamma-$Al_2O_3$ extrudates, CS331-4, was obtained from United Catalysts, Inc. (UCI) and was described by the manufacturer as 99.6% by weight $Al_2O_3$. It had a surface area of 175-275 $m^2/g$ and pore volume of 0.6 $cm^3/g$. A portion of this catalyst support was crushed and sieved and 2.87 grams (5.0 $cm^3$) loaded into a reactor tube as described above. In Examples 1 and 2 of Table 5, the temperature was 330˚C and 350˚C, respectively. At 350˚C, this unmodified gamma-$Al_2O_3$ catalyst which typically has only Lewis acidity, showed 63% conversion of PA and MMA, methacrylic acid (MAA), isobutyric acid (IBA) and MIB of 1.1%, 0.8%, 0.3% and 0.8%, respectively, as well as a MP (methyl propionate) selectivity of 92.0%. This is a very high selectivity to useful products of 95.0%. It has only a low methylation activity at the terminal carbon of 0.1 % butyric acid selectivity. At the lower temperature of 330˚C, the IBA and MAA selectivities increase to 0.7% and 1.6%, respectively, however, the MMA selectivity decreases to 0.6% and the byproducts, acetaldehyde and diethylketone, both increase significantly.

In Examples 1 and 2, it is shown that the desired products methyl propionate, methyl isobutyrate, methyl methacrylate, isobutyric acid and methacrylic acid are produced using gamma-$Al_2O_3$ catalyst with a combined selectivity at 350˚C of 95.0% at 63% PA conversion and 64% DME conversion. The DME/PA ratio was 0.82 and the DME/$O_2$ ratio was 3.8.

Examples 3, 4 and 5 *(gamma-$Al_2O_3$; stop $O_2$)*

In Examples 3, 4 and 5, the catalyst temperature was set at 330˚C then the flow of air was stopped and the product stream subsequently sampled at three 0.5-hour increments. The reaction conditions and catalytic results are shown in Table 5.

Examples 3, 4 and 5 demonstrate that the selectivity to methylation as indicated by IBA and MIB remains unchanged. The examples also show that the selectivity to MMA and MAA are substantially eliminated when oxygen is absent from the feedstock. Compared to Example 1 at 330˚C, the methyl propionate selectivity increased to 90-95% apparently because the yield loss to acetaldehyde (ACH) seen in Example 1 was completely eliminated.

Examples 3 to 5 illustrate that when $O_2$ is absent from the feed the dehydrogenated products, such as methyl methacrylate and methacrylic acid, are eliminated or substantially reduced in less than about 1 hour time on stream. The methylated products such as methyl isobutyrate and isobutyric acid are unaffected. The results also show that the byproduct acetaldehyde is dependent on oxygen concentration, a parameter which must be optimized. The catalyst has a selectivity to esterification that is greater than 90%.

TABLE 5

| **Example No.** | **1** | **2** | **3** | **4** | **5** |
|---|---|---|---|---|---|
| **Catalyst** | **g-Al203** | **g-Al203** | **g-Al203** | **g-Al203** | **g-Al203** |
| **Temperature, ˚C** | 330 | 350 | 330 | 330 | 330 |
| **GHSV, hr-1** | 920 | 920 | 920 | 920 | 920 |
| **Mol. Frac. DME** | 0.2063 | 0.2063 | 0.2063 | 0.2063 | 0.2063 |
| **Mol. Frac. PA** | 0.2513 | 0.2513 | 0.2513 | 0.2513 | 0.2513 |
| **Mol. Frac. 02*** | 0.0542 | 0.0542 | 0 | 0 | 0 |
| | **Conversion** | **Conversion** | **Conversion** | **Conversion** | **Conversion** |
| **% DME** | 56 | 64 | 65 | 44 | 51 |
| **% PA** | 43 | 63 | 36 | 34 | 49 |

(continued)

| | Selectivity | Selectivity | Selectivity | Selectivity | Selectivity |
|---|---|---|---|---|---|
| **%C, DME-based** | | | | | |
| CO | 4.4 | 10.0 | 0.7 | 0.0 | 0.0 |
| CH4 | 0.0 | 0.0 | 0.0 | 0.0 | 0.0 |
| CO2 | 7.7 | 7.2 | 1.0 | 4.4 | 3.0 |
| MeOH | 1.9 | 3.2 | 1.4 | 1.5 | 2.3 |
| methyl formate | 0.0 | 0.0 | 0.0 | 0.0 | 0.0 |
| **%C, PA-based** | | | | | |
| methyl propionate | 82.5 | 92.0 | 92.9 | 90.2 | 94.8 |
| methyl isobutyrate | 0.7 | 0.8 | 0.5 | 0.5 | 0.6 |
| acetone | 0.0 | 0.0 | 0.0 | 0.0 | 0.0 |
| methyl methacrylate | 0.6 | 1.1 | 0.3 | 0.0 | 0.0 |
| propanal | 0.0 | 0.0 | 0.0 | 0.0 | 0.0 |
| isobutyric acid | 0.7 | 0.3 | 0.7 | 0.6 | 0.5 |
| butyric acid | 0.0 | 0.1 | 0.0 | 0.0 | 0.0 |
| methacrylic acid | 1.6 | 0.8 | 1.7 | 0.1 | 0.1 |
| acrylic acid | 0.0 | 0.1 | 0.2 | 0.0 | 0.0 |
| acetaldehyde | 8.1 | 2.6 | 0.0 | 0.0 | 0.0 |
| methyl acetate | 0.3 | 1.4 | 0.6 | 0.5 | 0.7 |
| ethyl propionate | 0.0 | 0.1 | 0.0 | 0.0 | 0.0 |
| diethylketone | 3.5 | 1.7 | 2.5 | 7.2 | 2.9 |
| acetic acid | 2.0 | 0.9 | 0.7 | 0.9 | 0.4 |
| **Total Carbon Balance** | 89.7 | 101.8 | 90.2 | 97.1 | 88.6 |

* Balance is $N_2$, i.e., Mole Fraction ($DME+PA+O_2+N_2$) = 1.0

Example 6

2-methyl-1-butene (21g, 300 mmole) is added with stirring by a Teflon stirrer bar to a "nickel boride" slurry hydrogenation catalyst (37.5 mmol) in ethanol (175 ml). (The slurry hydrogenation catalyst is made separately by the reaction of sodium borohydride with aqueous nickel salts (Brown in J. Org. Chem. 1970, 35, 1900). The supernate from the catalyst preparation is decanted and the fine black granules are washed with ethanol and the ethanol is decanted. Ethanol (175 ml) is added to the fine black granules to prepare a slurry of the "nickel boride" hydrogenation catalyst.) The 2-methyl-1-butene and ethanol mixture is connected or added to a hydrogenator or Parr apparatus.

The system is purged with hydrogen and pressurized between 1 to 5 atm. with $H_2$. In approximately 30 minutes at 25˚C, the reaction is about 80% complete. After a total of 3 hours, the reaction is stopped and the hydrogenation catalyst is removed and the hydrogenated product is isolated by distillation (b.p. of 2-methyl butane is 30˚C).

Other catalysts can be used, such as Adams platinum oxide or palladium oxide. Both catalysts can be used using ethanol as solvent at the same hydrogenation temperature and hydrogen pressures. The reaction times will vary. In general for these types of hydrogenation catalysts, mono-substituted olefins are hydrogenated most rapidly and tri-substituted double bonds are hydrogenated more slowly.

## Claims

**1.** A process for providing a methyl group on an alpha-carbon adjacent to a hydrogenated electron withdrawing group, said process comprising:

providing a molecule containing the alpha-carbon and an electron withdrawing group;
reacting the molecule in a presence of an acid catalyst with dimethyl ether to substitute the methyl group on the alpha-carbon; and
hydrogenating the electron withdrawing group to provide the hydrogenated electron withdrawing group adjacent to the alpha-carbon substituted with the methyl group.

**2.** The process of claim 1, wherein the reacting comprises combining the molecule and the dimethyl ether in a vapor, liquid or slurry phase.

**3.** The process of claim 2, wherein the reacting is represented by at least one equation selected from the group consisting of:

(a) Eq. I $R'(CH_2)_nCH_2\text{-}EWG + CH_3OCH_3 \rightarrow R'(CH_2)_nCH(CH_3)\text{-}EWG + CH_3OH$

and

(b) Eq. II $R'(CH_2)_nCH_2\text{-}EWG\text{-}CH_2(CH_2)_mR'' + 4CH_3OCH_3 \rightarrow R'(CH_2)_nCH(CH_3)\text{-}EWG\text{-}CH_2(CH_2)_mR'' + R'(CH_2)_nCH_2\text{-}EWG\text{-}CH(CH_3)(CH_2)_mR''$ + $R'(CH_2)_nCH(CH_3)\text{-}EWG\text{-}CH(CH_3)(CH_2)_mR'' + 4CH_3OH$

and the hydrogenating is represented by at least one equation selected from the group consisting of:

(c) Eq. III $R'(CH_2)_nCH(CH_3)\text{-}EWG + H_2 \rightarrow R'(CH_2)_nCH(CH_3)\text{-}REWG$;

(d) Eq. IV $R'(CH_2)_nCH(CH_3)\text{-}EWG\text{-}CH(CH_3)(CH_2)_mR'' + H_2 \rightarrow R'(CH_2)_nCH(CH_3)\text{-}REWG\text{-}CH(CH_3)(CH_2)_mR''$;

(e) Eq. V $R'(CH_2)_nCH(CH_3)\text{-}EWG\text{-}CH_2(CH_2)_mR'' + H_2 \rightarrow R'(CH_2)_nCH(CH_3)\text{-}REWG\text{-}CH_2(CH_2)_mR''$;

and

(f) Eq. VI $R'(CH_2)_nCH_2\text{-}EWG\text{-}CH(CH_3)(CH_2)_mR'' + H_2 \rightarrow R'(CH_2)_nCH_2\text{-}REWG\text{-}CH(CH_3)(CH_2)_mR''$,

where R' is H when n is 0 to 18, otherwise R' is alkyl, alkene, EWG or aryl, and wherein: EWG is the electron withdrawing group; REWG is the hydrogenated electron withdrawing group; for Equations I and III, R' is H when n is 0 to 18 and alkyl, alkene, EWG or aryl when n > 18; and for Equations II and IV-VI, R" is H when m is 0 to 9 and alkyl, alkene, EWG or aryl when m > 9.

**4.** The process of claim 2, wherein the molecule is a member selected from the group consisting of an acid, an ester, a nitrile, a refinery olefin and a ketone.

**5.** The process of claim 2, wherein the molecule is a member selected from the group consisting of acetic acid, propionic acid, methyl acetate, methyl propionate, acetonitrile, propionitrile and acetone.

**6.** The process of claim 2, wherein the molecule is provided in a feedstock free of hydrogen.

**7.** The process of claim 2, wherein the molecule is provided in a feedstock free of oxidants.

**8.** The process of claim 2, wherein the electron withdrawing group is a member selected from the group consisting of carboxylic acids, carboxylic acid esters, nitriles, aromatic rings, ketones and olefins.

**9.** The process of claim 8, wherein the molecule is a member selected from the group consisting of an acid, an ester, a nitrile, an olefin and a ketone.

**10.** The process of claim 9, wherein the acid catalyst is a member selected from the group consisting of gamma-alumina, amorphous silica-alumina, a steam-treated zeolite, an acid washed clay, an alumina impregnated clay, and $MoO_3$ on gamma-alumina.

**11.** The process of claim 10, wherein the process is conducted without a basic catalyst.

**12.** The process of claim 2, wherein the acid catalyst is selected from the group consisting of Lewis acid catalysts, combined Lewis acid and Brönsted acid catalysts, and $MoO_3$ on gamma-alumina, and the reacting is conducted without a base catalyst.

**13.** The process of claim 2, wherein the electron withdrawing group is $-CO_2H$ or $-CO_2R$ and the reduced electron withdrawing group is $-CH_2OH$.

**14.** The process of claim 2, wherein the electron withdrawing group is -COR and the reduced electron withdrawing group is -CHOHR.

**15.** The process of claim 2, wherein the electron withdrawing group is -CN and the reduced electron withdrawing group is $-CH_2NH_2$.

**16.** The process of claim 2, wherein the electron withdrawing group is -C≡CR and the reduced electron withdrawing group is $-CH_2CH_2R$.

**17.** The process of claim 2, wherein the electron withdrawing group is

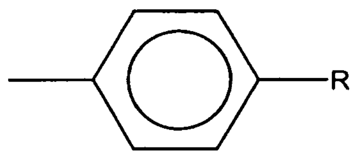

and the reduced electron withdrawing group is

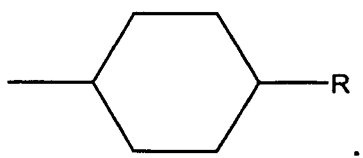

.

**18.** The process of claim 2, wherein the electron withdrawing group is -CH=CHR and the reduced electron withdrawing group is $-CH_2CH_2R$.

**19.** The process of claim 2, wherein the electron withdrawing group is -CH=CH- and the reduced electron withdrawing group is $-CH_2CH_2-$.

**20.** The process of claim 1, wherein more than one said methyl group is provided on more than one said alpha-carbon adjacent to more than one said hydrogenated electron withdrawing group.

**Patentansprüche**

**1.** Verfahren zum Bereitstellen einer Methylgruppe an einem α-Kohlenstoffatom, das an eine hydrierte, Elektronen abziehende Gruppe angrenzt, wobei das Verfahren folgendes umfaßt:

Bereitstellen eines Moleküls, das das α-Kohlenstoffatom und eine Elektronen abziehende Gruppe enthält;
Umsetzen des Moleküls mit Dimethylether in Gegenwart eines Säurekatalysators, so daß die Methylgruppe am α-Kohlenstoffatom substituiert wird; und
Hydrieren der Elektronen abziehenden Gruppe, so daß die hydrierte, Elektronen abziehende Gruppe bereitgestellt wird, die an das α-Kohlenstoffatom angrenzt, das mit der Methylgruppe substituiert ist.

**2.** Verfahren nach Anspruch 1, wobei die Umsetzung das Verbinden von dem Molekül und Dimethylether in einer Dampfphase, einer flüssigen oder einer Suspensionsphase umfaßt.

**3.** Verfahren nach Anspruch 2, wobei die Umsetzung mit zumindest einer Gleichung angegeben wird, die aus der folgenden Gruppe ausgewählt ist:

a) Gleichung I $R'(CH_2)_nCH_2\text{-EWG} + CH_3OCH_3 \rightarrow R'(CH_2)_nCH(CH_3)\text{-EWG} + CH_3OH$

und

b) Gleichung II $R'(CH_2)_nCH_2\text{-EWG-}CH_2(CH_2)_mR'' + 4CH_3OCH_3 \rightarrow R'(CH_2)_nCH(CH_3)\text{-EWG-}CH_2(CH_2)_mR'' + R'(CH_2)_nCH_2\text{-EWG-}CH(CH_3)(CH_2)_mR'' + R'(CH_2)_nCH(CH_3)\text{-EWG-}CH(CH_3)(CH_2)_mR'' + 4CH_3OH$

und das Hydrieren mit zumindest einer Gleichung angegeben wird, die aus der folgenden Gruppe ausgewählt ist:

c) Gleichung III $R'(CH_2)_nCH(CH_3)\text{-EWG} + H_2 \rightarrow R'(CH_2)_nCH(CH_3)\text{-REWG}$;

d) Gleichung IV $R'(CH_2)_nCH(CH_3)\text{-EWG-}CH(CH_3)(CH_2)_mR'' + H_2 \rightarrow R'(CH_2)_nCH(CH_3)\text{-REWG-}CH(CH_3)(CH_2)_mR''$;

e) Gleichung V $R'(CH_2)_nCH(CH_3)\text{-EWG-}CH_2(CH_2)_mR'' + H_2 \rightarrow R'(CH_2)_nCH(CH_3)\text{-REWG-}CH_2(CH_2)_mR''$;

und

f) Gleichung VI $R'(CH_2)_nCH_2\text{-EWG-}CH(CH_3)(CH_2)_mR'' + H_2 \rightarrow R'(CH_2)_nCH_2\text{-REWG-}CH(CH_3)(CH_2)_mR''$,

worin R' gleich H ist, wenn n gleich 0 bis 18 ist, ansonsten ist R' Alkyl, Alken, EWG oder Aryl, und wobei:

EWG die Elektronen abziehende Gruppe ist; REWG die hydrierte, Elektronen abziehende Gruppe ist; für die Gleichungen I und III ist R' gleich H, wenn n gleich 0 bis 18 ist, und Alkyl, Alken, EWG oder Aryl, wenn $n > 18$ ist; und für die Gleichungen II und IV bis VI ist R" gleich H, wenn m gleich 0 bis 9 ist, und Alkyl, Alken, EWG oder Aryl, wenn $m > 9$ ist.

**4.** Verfahren nach Anspruch 2, wobei das Molekül ein Molekül ist, das aus der Gruppe von einer Säure, einem Ester, einem Nitril, einem Raffinerieolefin und einem Keton ausgewählt ist.

**5.** Verfahren nach Anspruch 2, wobei das Molekül ein Molekül ist, das aus der Gruppe von Essigsäure, Propionsäure, Methylacetat, Methylpropionat, Acetonitril, Propionitril und Aceton ausgewählt ist.

**6.** Verfahren nach Anspruch 2, wobei das Molekül in einem wasserstoffreien Beschickungsmaterial bereitgestellt wird.

**7.** Verfahren nach Anspruch 2, wobei das Molekül in einem Beschickungsmaterial bereitgestellt wird, das frei von Oxidantien ist.

**8.** Verfahren nach Anspruch 2, wobei die Elektronen abziehende Gruppe eine Gruppe ist, die aus der Gruppe von Carbonsäuren, Carbonsäureestern, Nitrilen, aromatischen Ringen, Ketonen und Olefinen ausgewählt ist.

**9.** Verfahren nach Anspruch 8, wobei das Molekül ein Molekül ist, das aus der Gruppe von einer Säure, einem Ester, einem Nitril, einem Olefin und einem Keton ausgewählt ist.

**10.** Verfahren nach Anspruch 9, wobei der Säurekatalysator eine Verbindung ist, die aus der Gruppe von γ-Aluminiumoxid, amorphem Silciumdioxid-Aluminiumoxid, einem der Dampfbehandlung unterzogenen Zeolith, einem mit Säure gewaschenen Ton, einem mit Aluminiumoxid imprägnierten Ton und $MoO_3$ auf y-Aluminiumoxid ausgewählt ist.

**11.** Verfahren nach Anspruch 10, wobei das Verfahren ohne einen basischen Katalysator durchgeführt wird.

**12.** Verfahren nach Anspruch 2, wobei der Säurekatalysator aus der Gruppe von Lewis-Säure-Katalysatoren, kombinierten Lewis-Säure- und Brönsted-Säure-Katalysatoren und $MoO_3$ auf y-Aluminiumoxid ausgewählt ist und die Umsetzung ohne einen Basenkatalysator erfolgt.

**13.** Verfahren nach Anspruch 2, wobei die Elektronen abziehende Gruppe $-CO_2H$ oder $-CO_2R$ ist und die reduzierte, Elektronen abziehende Gruppe $-CH_2OH$ ist.

**14.** Verfahren nach Anspruch 2, wobei die Elektronen abziehende Gruppe -COR ist und die reduzierte, Elektronen abziehende Gruppe -CHOHR ist.

**15.** Verfahren nach Anspruch 2, wobei die Elektronen abziehende Gruppe -CN ist und die reduzierte, Elektronen abziehende Gruppe $-CH_2NH_2$ ist.

**16.** Verfahren nach Anspruch 2, wobei die Elektronen abziehende Gruppe $-C{\equiv}CR$ ist und die reduzierte, Elektronen abziehende Gruppe $-CH_2CH_2R$ ist.

**17.** Verfahren nach Anspruch 2, wobei die Elektronen abziehende Gruppe

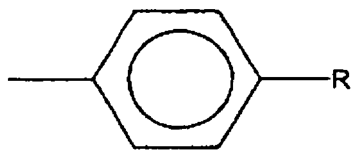

ist und die reduzierte, Elektronen abziehende Gruppe

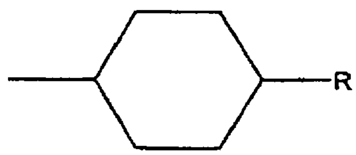

ist.

**18.** Verfahren nach Anspruch 2, wobei die Elektronen abziehende Gruppe -CH=CHR ist und die reduzierte, Elektronen abziehende Gruppe $-CH_2CH_2R$ ist.

**19.** Verfahren nach Anspruch 2, wobei die Elektronen abziehende Gruppe -CH=CH- ist und die reduzierte, Elektronen abziehende Gruppe $-CH_2CH_2-$ ist.

**20.** Verfahren nach Anspruch 1, wobei mehr als eine dieser Methylgruppen an mehr als einem dieser $\alpha$-Kohlenstoffatome vorgesehen wird, die an mehr als eine dieser hydrierten, Elektronen abziehenden Gruppen angrenzen.

## Revendications

**1.** Procédé permettant de placer un groupe méthyle sur un atome de carbone en alpha adjacent à un groupe attracteur d'électrons hydrogéné, ledit procédé comprenant les étapes consistant à :

fournir une molécule contenant l'atome de carbone en alpha et un groupe attracteur d'électrons ;
faire réagir la molécule en présence d'un catalyseur acide avec de l'éther diméthylique pour placer un substituant méthyle sur l'atome de carbone en alpha ; et
hydrogéner le groupe attracteur d'électrons pour obtenir le groupe attracteur d'électrons hydrogéné en position adjacente à l'atome de carbone en alpha portant le substituant méthyle.

**2.** Procédé selon la revendication 1, dans lequel la réaction comprend le fait de combiner la molécule et l'éther diméthylique en phase vapeur, liquide ou en suspension.

3. Procédé selon la revendication 2, dans lequel la réaction est représentée par au moins une équation choisie dans le groupe constitué par :

(a) Eq.I $R'(CH_2)_nCH_2\text{-EWG} + CH_3OCH_3 \rightarrow R'(CH_2)_nCH(CH_3)\text{-EWG} + CH_3OH$ et

(b) Eq.II $R'(CH_2)_nCH_2\text{-EWG-}CH_2(CH_2)_mR'' + 4CH_3OCH_3 \rightarrow R'(CH_2)_nCH(CH_3)\text{-EWG-}CH_2(CH_2)_mR'' + R'(CH_2)_nCH_2\text{-EWG-}CH(CH_3)(CH_2)_mR'' + R'(CH_2)_nCH(CH_3)\text{-EWG-}CH(CH_3)(CH_2)_mR'' + 4CH_3OH$

et l'hydrogénation est représentée par au moins une équation choisie dans le groupe constitué par :

(c) Eq. III $R'(CH_2)_nCH(CH_3)\text{-EWG} + H_2 \rightarrow R'(CH_2)_nCH(CH_3)\text{-REWG}$ ;

(d) Eq. IV $R'(CH_2)_nCH(CH_3)\text{-EWG-}CH(CH_3)(CH_2)_mR'' + H_2 \rightarrow R'(CH_2)_nCH(CH_3)\text{-REWG-}CH(CH_3)(CH_2)_mR''$ ;

(e) Eq. V $R'(CH_2)_nCH(CH_3)\text{-EWG-}CH_2(CH_2)_mR'' + H_2 \rightarrow R'(CH_2)_nCH(CH_3)\text{-REWG-}CH_2(CH_2)_mR''$;

et

(f) Eq. VI $R'(CH_2)_nCH_2\text{-EWG-}CH(CH_3)(CH_2)_mR'' + H_2 \rightarrow R'(CH_2)_nCH_2\text{-REWG-}CH(CH_3)(CH_2)_mR''$,

dans lesquelles R' représente H lorsque n a une valeur de 0 à 18, sinon R' représente un groupe alkyle, alcène, EWG ou aryle, et dans lesquelles EWG représente le groupe attracteur d'électrons ; REWG représente le groupe attracteur d'électrons hydrogéné ; pour les équations I et III, R' représente H lorsque n a une valeur de 0 à 18 et un groupe alkyle, alcène, EWG ou aryle lorsque n > 18 ; et pour les équations II et IV-VI, R" représente H lorsque m a une valeur de 0 à 9 et un groupe alkyle, alcène, EWG ou aryle lorsque m>9.

4. Procédé selon la revendication 2, dans lequel la molécule est un des éléments choisi dans le groupe constitué par un acide, un ester, un nitrile, une oléfine de raffinerie et une cétone.

5. Procédé selon la revendication 2, dans lequel la molécule est un des éléments choisi dans le groupe constitué par l'acide acétique, l'acide propionique, l'acétate de méthyle, le propionate de méthyle, l'acétonitrile, le propionitrile et l'acétone.

6. Procédé selon la revendication 2, dans lequel la molécule est fournie dans une charge d'alimentation sans hydrogène.

7. Procédé selon la revendication 2, dans lequel la molécule est fournie dans une charge d'alimentation sans oxydants.

8. Procédé selon la revendication 2, dans lequel le groupe attracteur d'électrons est un des éléments du groupe constitué par les acides carboxyliques, les esters d'acide carboxylique, les nitriles, les noyaux aromatiques, les cétones et les oléfines.

9. Procédé selon la revendication 8, dans lequel la molécule est un des éléments du groupe constitué par un acide, un ester, un nitrile, une oléfine et une cétone.

10. Procédé selon la revendication 9, dans lequel le catalyseur acide est un des éléments du groupe constitué par la gamma-alumine, la silice-alumine amorphe, une zéolite traitée à la vapeur d'eau, une argile lavée à l'acide, une argile imprégnée d'alumine, et $MoO_3$ sur de la gamma-alumine.

11. Procédé selon la revendication 10, dans lequel le procédé est réalisé sans catalyseur basique.

12. Procédé selon la revendication 2, dans lequel le catalyseur acide est choisi dans le groupe constitué par les catalyseurs acides de Lewis, les catalyseurs acides de Lewis et de Brönsted combinés, et $MoO_3$ sur de la gamma-alumine, et la réaction est réalisée sans catalyseur basique.

**13.** Procédé selon la revendication 2, dans lequel le groupe attracteur d'électrons est $-CO_2H$ ou $-CO_2R$ et le groupe attracteur d'électrons réduit est $-CH_2OH$.

**14.** Procédé selon la revendication 2, dans lequel le groupe attracteur d'électrons est -COR et le groupe attracteur d'électrons réduit est -CHOHR.

**15.** Procédé selon la revendication 2, dans lequel le groupe attracteur d'électrons est -CN et le groupe attracteur d'électrons réduit est $-CH_2NH_2$.

**16.** Procédé selon la revendication 2, dans lequel le groupe attracteur d'électrons est -C≡CR et le groupe attracteur d'électrons réduit est $-CH_2CH_2R$.

**17.** Procédé selon la revendication 2, dans lequel le groupe attracteur d'électrons est

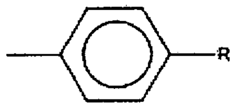

et le groupe attracteur d'électrons réduit est

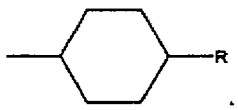

**18.** Procédé selon la revendication 2, dans lequel le groupe attracteur d'électrons est -CH=CHR et le groupe attracteur d'électrons réduit est $-CH_2CH_2R$.

**19.** Procédé selon la revendication 2, dans lequel le groupe attracteur d'électrons est -CH=CH- et le groupe attracteur d'électrons réduit est $-CH_2CH_2-$.

**20.** Procédé selon la revendication 1, dans lequel plus d'un desdits groupes méthyle est amené sur plus d'un desdits atomes de carbone en alpha adjacents à plus d'un desdits groupes attracteurs d'électrons hydrogénés.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4736062 A
- US 4761393 A
- US 4801571 A
- US 4845070 A
- US 4942258 A
- US 5710328 A
- US 5808148 A
- US 4442307 A
- US 3845155 A
- US 6329549 B
- EP 0111605 A
- US 6329549 B1

### Non-patent literature cited in the description

- **SPIVEY.** *Ind. Eng. Chem. Res.,* 1997, vol. 36 (11), 4600-4608
- *Ind. Eng. Chem. Res.,* 1997, vol. 36 (11), 4600-4608
- Symposium Syngas Conversion to Fuels and Chemicals. Div. Pet. Chem., Inc. American Chemical Society, 1999, 34-36
- *Catalysis Reviews, Sci. Eng.,* 1998, vol. 40 (1&2), 1-38
- **AI.** *Applied Catalysis A: General,* 1994, vol. 109, 135-146
- *Journal of Catalysis,* 1986, vol. 98, 401-410
- **J. CATAL.** *J. Catal.,* 1987, vol. 107, 201-208
- **WATZENBERGER.** *Journal of Catalysis,* 1990, vol. 124, 247-258
- **NISHIMURA.** Handbook of Heterogeneous Catalytic Hydrogenation for Organic Synthesis. John Wiley and Sons, Inc, 2001
- **STULL et al.** The Chemical Thermodynamics of Organic Compounds. John Wiley and Sons, Inc, 1969